(19) Europäisches Patentamt
European Patent Office
Office européen des brevets

(11) Publication number: **0 161 932 B1**

(12) **EUROPEAN PATENT SPECIFICATION**

(45) Date of publication of patent specification: **13.04.88**

(51) Int. Cl.⁴: **C 07 C 149/40,** C 07 D 337/14

(21) Application number: **85303365.2**

(22) Date of filing: **13.05.85**

(54) **Preparation of carboxyphenylthiophenylacetic acids.**

(30) Priority: **14.05.84 US 610064**

(43) Date of publication of application:
**21.11.85 Bulletin 85/47**

(45) Publication of the grant of the patent:
**13.04.88 Bulletin 88/15**

(84) Designated Contracting States:
**CH DE FR GB IT LI NL**

(56) References cited:
**EP-A-0 052 912**
**FR-A-1 270 929**

(73) Proprietor: **MERCK & CO. INC.**
**126, East Lincoln Avenue P.O. Box 2000**
**Rahway New Jersey 07065-0900 (US)**

(72) Inventor: **Verhoeven, Thomas R.**
**106 Oak Lane**
**Cranford New Jersey 07016 (US)**

(74) Representative: **Crampton, Keith John Allen et al**
**D YOUNG & CO 10 Staple Inn**
**London WC1V 7RD (GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to the preparation of dibenzo[b,f]thiepins and their derivatives. More particularly, it is concerned with the preparation of certain carboxyphenylthiophenylacetic acids, which are used as intermediate compounds in the synthesis of such dibenzothiepins, which are prostaglandin antagonists. Examples of prostaglandin antagonists that may be prepared from the intermediate compounds made using the methods of the present invention are disclosed in U.S. Patent Specification US—A—4,237,160, U.S. Patent Application No. 396,452 and European Patent Specification EP—A—0011067.

In one embodiment of the present invention, dibenzo[b,f]thiepin and its derivatives are prepared by a process that includes direct carboxylation of an ortho-toluyl-aryl sulfide to introduce a phenylacetic acid side chain. This transformation generally functions as the crucial step in the synthesis of dibenzo[b,f]thiepin compounds.

Prior syntheses of compounds containing the dibenzo[b,f]thiepin skeleton (see, for example, U.S. Patent Specification US—A—4,237,160 and M. Rajsner *et al.,* Collection of Czechoslov. Chem. Commun., *42,* 3079—3093 (1977) suffer from the non-availability and/or expensive nature of functionalized ortho-halo-phenylacetic acid derivatives required as starting materials. The preparation of these starting materials is inefficient and multi-step. The present invention obviates their use and allows the use of readily available ortho-halo-toluic acid derivatives in a single high-yielding carboxylation step, which replaces the low-yielding multi-step procedure of the prior art.

In the process of the present invention, and unlike in previous methods, the phenylacetic acid side chain is prepared after the diaryl sulfide linkage has been established. This introduces a greater flexibility in the synthesis of the dibenzo[b,f]thiepin skeleton and permits of the use of less costly and commercially available starting materials. This direct carboxylation approach establishes the phenylacetic acid side chain in a single high-yielding step.

In one of its embodiments, a process for preparing a compound of the formula I:

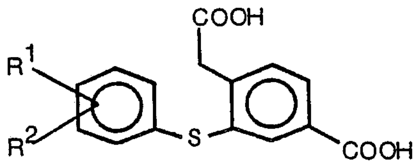

I

in which each of $R^1$ and $R^2$, independently of the other, is a hydrogen or halogen (i.e. fluorine, chlorine, bromine or iodine) atom or a $C_{1-4}$ alkyl, $C_{1-4}$ alkoxy, $C_{1-4}$ alkylthio, $C_{1-4}$ alkylsulfinyl, $C_{1-4}$ alkyl sulfonyl, trifluoromethyl, trifluoromethylthio, cyano, nitro, di($C_{1-4}$ alkyl)amino, carboxy, or phenyl-($C_{1-4}$ alkyl) radical, the phenylalkyl radical optionally having halogen, nitro or $C_{1-4}$ alkyl substitution in the phenyl ring, comprises treating a dianion of the formula II:

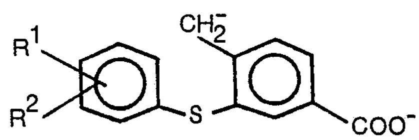

II

in which $R^1$ and $R^2$ are as defined above, with carbon dioxide in an inert solvent at a temperature of preferably −40 to 0°C, particularly −20°C. The pressure is preferably atmospheric but a higher pressure could be used. The inert solvent is preferably an ether such as tetrahydrofuran, 1,2-dimethoxyethane(glyme), bis(2-methoxyethyl)ether (diglyme) or ethyl ether, particularly a 20:1 mixture by volume of tetrahydrofuran and 1,3-dimethylimidazolidinone.

Each of $R^1$ and $R^2$, independently of the other, is preferably a hydrogen or halogen atom.

The aforementioned dianion may be formed from a compound of the formula III:

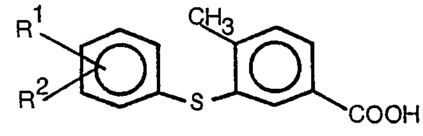

III

in which $R^1$ and $R^2$ are as defined above, by initially forming a carboxylate salt with a base, for example, sodium hydride,, followed by deprotonation. Deprotonation may be accomplished with an alkali metal dialkylamide base (for example, lithium diethylamide), an alkylamide base (for example, ethyl amide or isopropyl amide), or an alkali metal amide base, (for example, lithium or sodium amide). The preferred base is lithium diisopropylamide. The preferred temperature is −40°C to −10°C, particularly about −20°C. The pressure is preferably atmospheric, but a higher pressure could be used. The solvent should be inert

and is preferably an ether such as tetrahydrofuran, 1,2-dimethoxyethane (glyme), bis (2-methoxyethyl)ether (diglyme) or ethyl ether, particularly a 20:1 by volume mixture of tetrahydrofuran and 1,3-dimethylimidazolidinone. Preferably, the dianion prepared from the compound of Formula III is not isolated and the dianion of Formula II is immediately converted to the desired compound of Formula I.

In view of the fact that the carboxylation reaction may be difficult when $R^1$ and $R^2$ are bromine, iodine, alkythio, alkyl sulfinyl, alkyl sulfonyl or nitro, compounds of Formula II and the compounds of the Formula III from which they are prepared having such substituents are not preferred as starting materials.

The process of the present invention is also applicable to compounds in which each of $R^1$ and $R^2$, independently of the other, is amino, $C_{1-4}$ alkanoyl, hydroxyl, thio, $C_{1-4}$ alkylamino, $C_{1-4}$ hydroxyalkyl, mercapto, and phenyl-$C_{1-4}$ alkyl) in which the phenyl ring has an amino or hydroxy substituent. For such compounds, however, these functional groups should be protected prior to the carboxylation reaction.

The following reaction scheme showing the synthesis of 2-(3′-fluorophenylthio)-4-carboxyphenylacetic acid, which is an intermediate compound in the production of *S*-7-fluorodibenzo[b,f]thiepin-3-carboxylic acid-5-oxide, illustrates the importance of the process of the present invention:

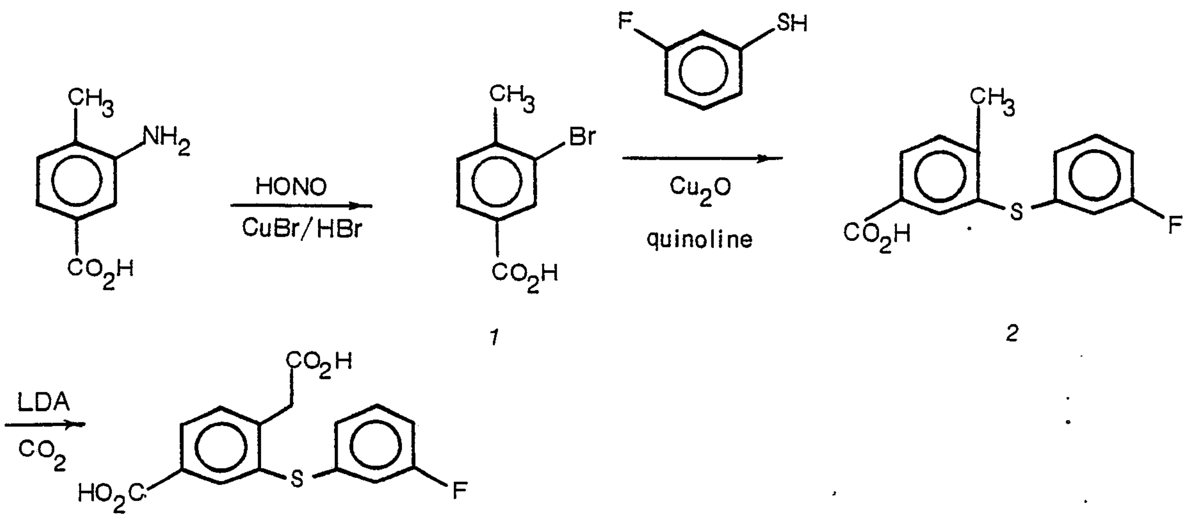


1

2

4

The initial step in the above synthesis involves diazotization and bromination of 3-amino-4-methylbenzoic acid, which proceeds in 95% yield. Reaction of this aryl bromide (*1*) with m-fluorothiophenol produces sulfide *2* in 89% yield. This reaction involves the *in situ* formation of 3-fluorophenylthiocopper (ArSCu) with cuprous oxide which subsequently couples with the aryl bromide at 180—190°C. Incorporation of the diaryl sulfide linkage at this early stage in the process results in improved yields and elimination of side reactions compared to the prior process. Use of the readily available aryl bromide *1* obviates the use of iodide or bromide *3*, an economically unattractive intermediate compound in the prior synthesis.

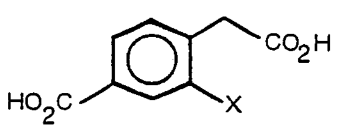

X = Br, I

3

The crucial carboxylation of *2* is accomplished in a single step via its dianion. The dianion is generated by initial formation of the carboxylate salt with sodium hydride with final benzylic deprotonation being accomplished with lithium diisopropylamide at −20°C. Treatment with carbon dioxide produces diacid *4* in 92% yield. This direct carboxylation approach establishes the phenylacetic acid side chain in a single high-yielding step, thus eliminating the previous three-step sequence of bezylic photo-bromination, cyanide displacement and hydrolysis (overall yield 30—40%). Ring closure may be effected by methods disclosed in U.S. Patent 4,237,160.

The following reaction scheme shows a similar synthesis of 4-carboxy-2-phenylthiophenylacetic acid *6* which is an intermediate in the preparation of 3-hydroxymethyldibenzo[b,f]thiepin-5,5-dioxide:

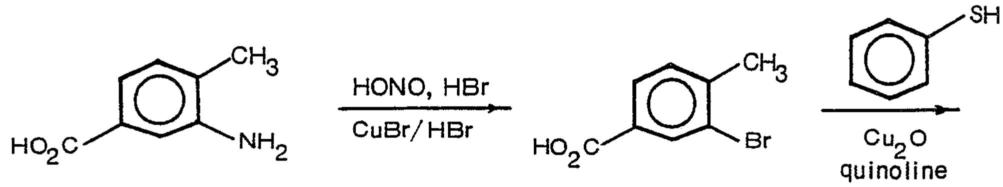


1

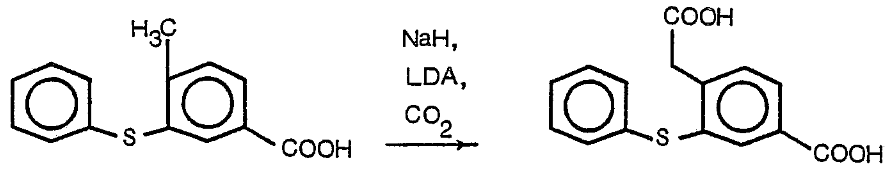


5 6

Reaction of 3-bromo-4-methylbenzoic acid *1* with thiophenol and cuprous oxide in quinoline at 180°C produces 3-phenylthio-4-methylbenzoic acid *5* in 95—96% yield. Generation of the dianion of *5* is accomplished by sequential treatment with sodium hydride and then lithium diisopropylamide in a solvent mixture of tetrahydrofuran and 1,3-dimethylimidazolidinone at −20°C. Carboxylation at −15°C with carbon dioxide at a pressure of one atmosphere produces the diacid *6* in 90% yield.

The following Examples are illustrative of the present invention:

Example 1

Preparation of 2-(3'-Fluorophenylthio)-4-carboxyphenylacetic acid

Step A: 3-Bromo-4-methylbenzoic acid

A 22-liter 3-necked, round-bottom flask, fitted with an overhead stirrer, thermometer, and a 2-liter additional funnel was charged with water (6.4 liters) and 3-amino-4-methylbenzoic acid (2.1 kg, 13.89 mole) to produce a thick slurry. While cooling the mixture in an ice/water bath, concentrated hydrochloric acid (1.76 liters) was added over a 5-minute period. The internal temperature rose to 35°C during this addition. The mixture was recooled to −3°C with an aqueous ethanol/dry ice bath and a solution of sodium nitrite (1006 g, 14.58 mole) dissolved in water (1.4 liters) was added over a 1-hour period, maintaining the temperature between −3° and 0°C. During the final stages of the addition, gas evolution and consequent foaming were observed. Care should be exercised to maintain the cooling bath temperature at no lower than −10°C. Below that temperature significant precipitation of the diazonium salt occurs thereby hampering its transferral from the reaction vessel.

Meanwhile, a 30 U.S. gallon (114 liter) polypropylene vat, equipped with an overhead stirrer, and a 3 liter jacketed brine-cooled addition funnel was charged with 48% hydrobromic acid (19 liters) and cuprous bromide (3.52 kg, 24.53 mole) and heated to 30—35°C in a water bath. The cold diazonium salt solution was transferred in portions to the brine-cooled addition funnel (to maintain the temperature of the diazonium hydrochloride solution at 0°C) and added to the vigorously stirred cuprous bromide solution over about 1.5 hours, maintaining a reaction temperature of 35—40°C and adding diethyl ether periodically in 100 to 300 ml portions with a total volume of about 800 ml being added, in order to reduce the volume of foam caused by nitrogen evolution. Nitrogen evolution subsided and the mixture was stirred at ambient temperature overnight.

The product was recovered from the reaction mixture by suction filtration. The filter cake was sucked completely dry because addition of water to the unfiltered reaction mixture precipitates highly water-insoluble copper salts which it is difficult to remove. The filter cake was washed with sufficient water until neutral (as tested with alk-acid paper) and the product was air-dried at 70—75°C overnight to provide a tan solid (2811 g, 94.1%), mp 203—205°C.

Recrystallization of a sample from aqueous ethanol provided cream-colored needles, mp 206—207.5°C.

Analysis of the product (prior to recrystallization) by HPLC (reverse phase, Altex, ultrasphereoctyl, 5 μm, 25 cm × 4.6 mm I.D., $CH_3CN$:$H_2O$, 50:50 v/v with 0.1% $H_3PO_4$, flow rate = 2.0 ml/minute) indicated an impurity (retention time = 2.0 minutes) of 8—10% (based on UV absorbance at 254 nm). The retention time of the product is 4.2—4.3 minutes. This impurity is believed to be 3-hydroxy-4-methyl benzoic acid based upon expected competitive side processes.

STEP B: 3-(3'-Fluorophenylthio)-4-methylbenzoic acid

A 22-liter 3-necked round bottom flask, fitted with an overhead stirrer, thermometer and distillation head was placed in a heating mantle and charged with quinoline (6 liters), 3-bromo-4-methyl-benzoic acid (2.725 kg, 12.67 mole), m-fluorothiophenol (1.70 kg, 13.28 mole) and cuprous oxide (958 g, 6.69 mole). Due to its pervasive stench, the m-fluorothiophenol was transferred in a fume hood. The reaction of 3-bromo-4-methylbenzoic acid and m-fluorothiophenol with quinoline was exothermic causing a temperature rise to

75°C. The red slurry was placed under a nitrogen blanket and heated with stirring to 180—190°C over a 1-hour period collecting the water as it distilled. When the internal temperature reached 110°C, the mixture became very thick and distillation of water commenced. This thickening was caused by the formation and precipitation of (m-fluorothiophenyl)copper. Continued heating slowly redissolved this precipitate with the mixture becoming completely homogeneous at a temperature of 150°C. This temperature range was maintained for 0.5 hour. The progress of the coupling may be conveniently monitored by reverse phase HPLC (Altex, ultrasphere-octyl, 5 µm, 25 cm × 4.6 mm I.D., 50:50, volume/volume, $CH_3CN$:$H_2O$ with 0.1% $H_3PO_4$, 2 ml/minute). Retention time; 3-bromo-4-methylbenzoic acid: 4.2—4.3 minute, 3-(3'-fluorophenylthio)-4-methylbenzoic acid: 9.7—10.0 minute. The black mixture was allowed to cool to 110°C and then transferred to a stirred solution of 6N hydrochloric acid (28 liters).

After stirring overnight, the crude product was collected by suction filtration and washed with 6N hydrochloric acid (19 liters), and then with water until the filtrate was neutral (alk-acid paper). The filter cake was washed with five 1-liter portions of isopropanol and air-dried overnight at 40°C to provide the title compound as a dark-tan solid (2785 g, 83.8%) mp 154—158°C. Recrystallization of a sample from toluene yielded white needles, mp 162—163°C. Analysis for $C_{14}F_{11}FO_2S$,

calculated: C: 64.11; H: 4.20; S: 12.22; F: 7.24.
found: C: 64.25; H: 4.23; S: 12.29; F: 7.38.

Quantitative HPLC assay of this material (prior to recrystallization) using column conditions as described above in this paragraphs, indicated a product purity of 95%.

The isopropanol filtrate was concentrated to dryness, dissolved in acetone (2 liter) and treated with charcoal (82 g) for 30 minutes. Filtration through Celite and concentration under vacuum provided a dark solid which was crystallized from hot toluene (2 liter) to yield a second crop of product as tan needles (305 g, 9.2%) mp 140—144°C. Both crops were suitable for use directly in the subsequent transformation.

If desired, an alternative procedure may be used to obtain material of higher melting point. This involves azeotropically drying the water-washed filter cake with toluene and subsequent crystallization from a minimum volume of hot toluene (approximately 200 g/liter). When this modification was performed on a reaction involving 1660 g (7.72 mole) of 3-bromo-4-methylbenzoic acid a first crop of 1631 g (08.8%), mp 161.5—163°C and a second crop of 124 g (6.1%), mp 158—161°C were obtained for a combined yield of 86.9%.

STEP C: 2-(3'-Fluorophenylthio)-4-carboxyphenylacetic acid

In this step, all glassware was dried via a vacuum purge technique under nitrogen, with all manipulations carried out under an inert atmosphere and the solvents (tetrahydrofuran, 1,3-dimethyl 2-imidazolidinone and diisopropylamine) were all dried over 4 Angstrom (400 pm) sieves prior to use.

A dry, 3-necked, 12-liter round bottom flask, equipped with an overhead stirrer, condenser, calibrated addition funnel, thermometer and nitrogen inlet was charged with sodium hydride (202 g, 50% oil dispersion, 4.12 mole), tetrahydrofuran (300 ml) and 1,3-dimethyl-2-imidazolidinone (DMI) (525 ml). A slight out-gassing and color change occurs upon addition of DMI to the sodium hydride. 3-(3'-Fluorophenylthio)-4-methylbenzoic acid (1050 g, 4.01 mole) was dissolved in tetrahydrofuran (2300 ml) under nitrogen (endothermic) and slowly added over 2 hours to the sodium hydride slurry with vigorous stirring. Addition at this rate maintained the internal temperature between 55—60°C. Hydrogen evolution subsided and the mixture was allowed to cool slowly. The color of the reaction mixture at this stage may vary from golden yellow to black depending on the quality of the starting sulfide.

Meanwhile, a dry 3-necked 22-liter round-bottom flask, equipped with an additional funnel, thermometer, nitrogen inlet and overhead stirrer was charged with tetrahydrofuran (8.0 l) and diisopropylamine (725 ml, 5.17 mole). The source and quality of the THF used here is crucial. Fisher reagent grade tetrahydrofuran provided a clear yellow solution of lithium diisopropylamide. An inferior tetrahydrofuran might produce black-coloured mixtures of lithium diisopropylamide, unsuitable for use in this reaction. The integrity of the THF-diisopropylamine solution may be tested by treating a sample with n-butyllithium prior to proceeding with the entire batch.

The stirred solution was cooled to −30°C in an acetone/dry ice bath and n-butyllithium (3125 ml, 5.06 mole, 1.62 *M* in hexane) was added over 15 minutes. n-Butyllithium is commercially available in 5 U.S. gallon (19-liter) steel cylinders from Lithium Corporation of America and may be conveniently dispensed directly into the calibrated addition funnel immediately prior to its introduction to the reaction vessel. The temperature rose to −10°C and a clear yellow solution resulted. The solution was re-cooled to −40°C and the earlier prepared sulfide solution was added over 15 minutes, producing an opaque mixture. The cooling bath was removed, and the temperature allowed to rise to −20°C. On a large scale, the otherwise slow warm-up may be hastened by immersing the reaction vessel in cold water. Carbon dioxide (bone dry gas) was rapidly bubbled through the reaction mixture with vigorous stirring for 1.5 hours. The opaque mixture decolorized producing a cream-coloured precipitate.

Isopropanol (200 ml) was carefully added to consume any excess of sodium hydride or lithium diisopropylamide present, then 1 liter of water was carefully added over about 15 minutes. The reaction mixture darkened and layer separation occured. The mixture was transferred to a large extractor, diluted with an additional 3.5 l of water and stirred for 15 minutes. The lower aqueous layer was removed and the organic phase washed with 5% aqueous sodium hydroxide (1.6 liters). The total aqueous extracts were

combined and acidified with vigorous stirring with concentratesd hydrochloric acid (approximately 1.5 liters). The product separated as a solid. If oiling occurred, stirring was continued and 4 to 5 liters of hexane was added to induce solidification. After 2 hours of vigorous agitation the mixture was filtered, washed with water and air dried at 40°C to give a cream-colored powder, (1130 g, 92%), mp 200—203°C.

Recrystallization of a sample from aqueous isopropanol produced white needles, mp 207—207.5°C.

Analysis for $C_{15}H_{11}FSO_4$:

calculated: C: 58.81; H: 3.62; S: 10.47; F: 6.20.
found: C: 58.84; H: 3.57; S: 10.52; F: 6.38.

Quantitative HPLC analysis (reverse-phase, Altex, ultrasphere-octyl, 5 microns, 25 cm × 4.6 mm I.D., $CH_3CN:H_2O$, 40:60 v/v with 0.1% $H_3PO_4$, 2 ml/minute, 254 nm) indicated a purity of 87—89%.

Example 2

4-carboxy-2-phenylthiophenylacetic acid

Step A: 4-Methyl-3-phenylthiobenzoic acid

A 22-liter 3-necked round-bottom flask, fitted with an overhead stirrer, thermometer and distillation head was placed in a heating mantle and charged with quinoline (6 liters), 3-bromo-4-methylbenzoic acid (2.75 kg, 12.79 mole), thiophenol (17.5 kg, 15.22 mole) and cuprous oxide (958 g, 6.7 mole). The red slurry was placed under a nitrogen blanket and heated with stirring to 180—200°C over a 1 hour period collecting the water as it distils. This temperature was maintained for 0.5 hours. The mixture was cooled to 110°C and then transferred to a stirred solution of 6N hydrochloric acid. After overnight stirring, the product was collected by suction filtration, washed with 6N hydrochloric acid (19 liters) and then with water until neutral (alk-acid paper). The filter cake was washed with isopropanol and dried at 40°C *in vacuo* to yield the title compound as a tan solid (2.97 kg, 95% yield, 91% purity).

Step B: 4-Carboxy-2-phenylthiophenylacetic acid

A dry 3-necked, 12-liter round-bottom flask, equipped with an overhead stirrer, condenser, calibrated addition funnel, thermometer and nitrogen inlet was charged with sodium hydride (202 g, 50% oil disperson, 4.12 mole), tetrahydrofuran (300 ml) and 1,3-dimethyl-2-imidazolidinone (DMI) (525 ml). 3-Phenylthio-4-methylbenzoic acid (980 g, 4.01 mole) was dissolved in tetrahydrofuran (2300 ml) under nitrogen (endothermic) and slowly added over 2 hours to the sodium hydride slurry with vigorous stirring. Addition at this rate maintained the internal temperature between 55 and 60°C. Hydrogen evolution subsided and the mixture was allowed to cool slowly.

Meanwhile, a dry 3-necked 22-liter round-bottom flask, equipped with an addition funnel, thermometer, nitrogen inlet and overhead stirrer was charged with tetrahydrofuran (8.0 l) and diisopropylamine (725 ml, 5.17 mole). The stirred solution was cooled to −30°C and n-butyllithium (3125 ml, 5.06 mole, 1.62 *M* in hexane) was added over 15 minutes. The temperature increased to −10°C and a clear yellow solution resulted. The solution was re-cooled to −40°C and the earlier prepared sulfide solution was added over 15 minutes, producing an opaque mixture. The cooling bath was removed and the temperature allowed to rise to −20°C and aged for 10 minutes. Carbon dioxide (bone dry gas) was passed over the surface of the reaction mixture with vigorous stirring for 1.5 hours. The opaque mixture decolorized producing a cream-colored precipitate.

Isopropanol (200 ml) was carefully added to consume any excess of sodium hydride or lithium diisopropylamide present, then 1 liter of water was carefully added over about 15 minutes. The reaction mixture darkened and layer separation occured. The mixture was transferred to a large extractor, diluted with an additional 3.5 l of water and stirred for 15 minutes. The lower aqueous layer was removed and the organic phase washed with 5% aqueous sodium hydroxide (1.6 liters). The total aqueous extracts were combined and acidified with vigorous stirring with concentrated hydrochloric acid (approximately 1.5 liters). The product separated as a solid. If oiling occurs, stirring should be continued and 4 to 5 liters of hexane added to induce solidification. After 2 hours of vigorous agitation, the mixture was filtered, washed with water and air dried at 40°C to give a tan to cream-colored powder, (1060 g, 92% yield, 92% purity).

**Claims**

1. A method of preparing a compound of the formula I:

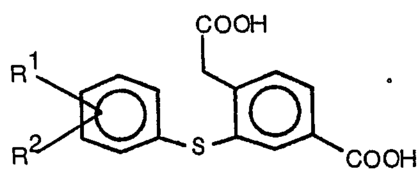

I

in which each of $R^1$ and $R^2$, independently of the other, is a hydrogen or halogen atom or a $C_{1-4}$ alkyl, $C_{1-4}$ alkoxy, $C_{1-4}$ alkylthio, $C_{1-4}$ alkylsulfinyl, $C_{1-4}$ alkyl sulfonyl, trifluoromethyl, trifluoromethylthio, cyano, nitro, di($C_{1-4}$ alkyl)amino, carboxy, or phenyl-($C_{1-4}$ alkyl) radical, the phenylalkyl radical optionally having

halogen, nitro or $C_{1-4}$ alkyl substitution in the phenyl ring, that comprises treating a dianion of the formula II:

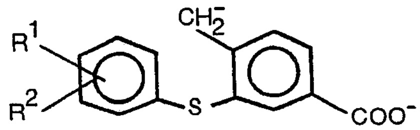

II

in which $R^1$ and $R^2$ are as defined above, with carbon dioxide in an inert solvent.

2. A method according to Claim 1, in which the temperature of the reaction medium is maintained at −40°C to 0°C.

3. A method according to Claim 2, in which the temperature is about −20°C.

4. A method according to any one of Claims 1 to 3 carried out at atmospheric pressure.

5. A method according to any one of the Claims 1 to 4 in which the solvent is an ether.

6. A method according to any one of Claims 1 to 4 in which the solvent is tetrahydrofuran, 1,2-dimethoxyethane, bis(2-methoxyethyl) ether, ethyl ether or a 20:1 mixture by volume of tetrahydrofuran and 1,3-dimethylimidazolidinone.

7. A method according to any one of Claims 1 to 6 in which each of $R^1$ and $R^2$, independently of the other, is a hydrogen or halogen atom.

8. A method according to Claim 7 in which $R^1$ is hydrogen and $R^2$ is halogen.

9. A method according to Claim 8 in which $R^1$ is hydrogen and $R^2$ is fluorine.

10. A method according to Claim 9 in which the fluorine is *meta* to the position of attachment of the (dicarboxyphenyl) thio radical to the left-hand ring in Formula I.

11. A method according to Claim 7 in which $R^1$ and $R^2$ are both hydrogen.

12. A method according to any one of Claims 1 to 11 in which the dianion is formed from a compound of the formula III:

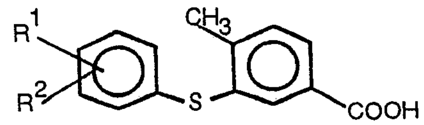

III

in which $R^1$ and $R^2$ are as defined in Claim 1 by initially forming a carboxylate salt with a base followed by deprotonation under conditions as set out in any one of Claims 2 to 6.

13. A method according to Claim 12 in which the deprotonation is accomplished with an alkali metal dialkylamide base, an alkylamide base or an alkali metal amide base.

14. A method of preparing a dibenzo[b,f]thiepin compound, characterized in that an intermediate carboxyphenylthiophenylacetic acid compound of the formula I set forth in Claim 1 is prepared by a method as claimed in any one of the preceding claims and then converted by known means to the desired dibenzo[b,f]thiepin.

**Patentansprüche**

1. Verfahren zur Herstellung einer Verbindung der Formel I:

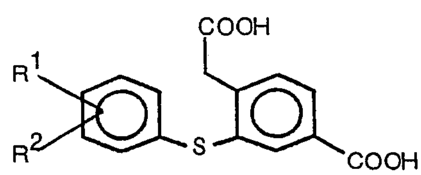

I

worin jedes von $R^1$ und $R^2$ unabhängig von dem anderen ein Wasserstoff- oder Halogenatom oder ein $C_{1-4}$Alkyl-, $C_{1-4}$Alkoxy-, $C_{1-3}$Alkylthio-, $C_{1-4}$Alkylsulfinyl-, $C_{1-4}$Alkylsulfonyl-, Trifluormethyl-, Trifluormethylthio-, Cyano-, Nitro-, Di($C_{1-4}$alkyl)amino-, Carboxy- oder Phenyl($C_{1-4}$alkyl)-Rest ist, wobei der Phenylalkylrest gegebenenfalls mit Halogen, Nitro oder $C_{1-4}$Alkyl im Phenylring substituiert ist, durch Behandeln eines Dianions der Formel II:

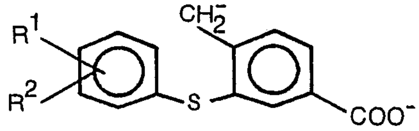

II

worin $R^1$ und $R^2$ wie oben definiert sind, mit Kohlendioxid in einem inerten Lösungsmittel.

2. Verfahren nach Anspruch 1, worin die Temperatur des Reaktionsmediums bei −40°C bis 0°C gehalten wird.

3. Verfahren nach Anspruch 2, worin die Temperatur etwa −20°C ist.

4. Verfahren nach einem der Ansprüche 1 bis 3, durchegeführt bei atmosphärischem Druck.

5. Verfahren nach einem der Ansprüche 1 bis 4, worin das Lösungsmittel ein Ether ist.

6. Verfahren nach einem der Ansprüche 1 bis 4, worin das Lösungsmittel Tetrahydrofuran, 1,2-Dimethoxyethan, Bis-(2-methoxyethyl)ether, Ethylether oder eine 20:1-Volumenmischung von Tetrahydrofuran und 1,3-Dimethylimidazolidinon ist.

7. Verfahren nach einem der Ansprüche 1 bis 6, worin jedes von $R^1$ und $R^2$ unabhängig von dem anderen ein Wasserstoff- oder Halogenatom ist.

8. Verfahren nach Anspruch 7, worin $R^1$ Wasserstoff ist und $R^2$ Halogen ist.

9. Verfahren nach Anspruch 8, worin $R^1$ Wasserstoff ist und $R^2$ Fluor ist.

10. Verfahren nach Anspruch 9, worin das Fluor *meta*-ständig zur Stellung der Anbindung des (Dicarboxyphenyl)thiorestes an den linksseitigen Ring in der Formel I ist.

11. Verfahren nach Anspruch 7, worin $R^1$ und $R^2$ beide Wasserstoff sind.

12. Verfahren nach einem der Ansprüche 1 bis 11, worin das Dianion aus einer Verbindung der Formel III gebildet wird.

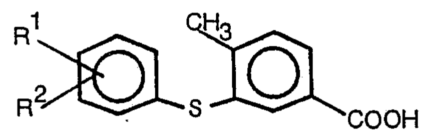

III

worin $R^1$ und $R^2$ wie in Anspruch 1 definiert sind, durch anfängliche Bilding eines Carboxylatsalzes mit einer Base, gefolgt von der Deprotonierung unter den in einem der Ansprüche 2 bis 6 genannten Bedingungen.

13. Verfahren nach Anspruch 12, worin die Deprotonierung durch eine Alkalimetalldialkylamidbase, eine Alkylamidbase oder eine Alkalimetallamidbase bewirkt wird.

14. Verfahren zur Herstellung eine Dibenzo[b,f]thiepinverbindung, dadurch gekennzeichnet, dass eine intermediäre Carboxyphenylthiophenylessigsäureverbindung der Formel I, wie in Anspruch 1 genannt, nach einem Verfahren, wie in einem der vorstehenden Ansprüche beansprucht, hergestellt wird und dann nach bekannten Verfahren in das erwünschte Dibenzo[b,f]thiepin umgewandelt wird.

**Revendications**

1. Procédé pour la préparation d'un composé de formule I:

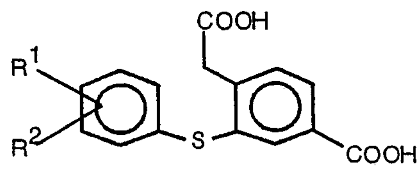

I

dans laquelle $R^1$ et $R^2$ représentent chacun, indépendamment l'un de l'autre, un atome d'hydrogène ou d'halogène ou un radical alkyle en $C_{1-4}$, alcoxy en $C_{1-4}$, (alkyl en $C_{1-4}$)-thio, (alkyl en $C_{1-4}$)-sulfinyle, (alkyl en $C_{1-4}$)- sulfonyle, trifluorométhyle, trifluorométhylthio, cyano, nitro, di(alkyl en $C_{1-4}$)-amino, carboxy ou phényl-(alkyle en $C_{1-4}$), le noyau phényle du radical phénylalkyle étant éventuellement substitué par un atome d'halogène, un radical nitro ou alkyle en $C_{1-4}$, qui comprend le traitement d'un dianion de formule II:

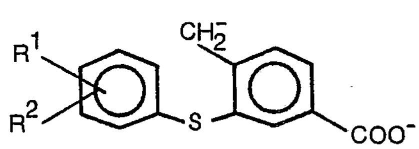

II

dans laquelle $R^1$ et $R^2$ sont tels que définis ci-dessus, avec du gaz carbonique dans un solvant inerte.

2. Procédé selon le revendication 1, dans lequel on maintient la température du milieu de réaction entre −40°C et 0°C.

3. Procédé selon la revendication 2, dans lequel la température est d'environ −20°C.

4. Procédé selon l'une quelconque des revendications 1 à 3, effectué à la pression atmosphérique.

5. Procédé selon l'une quelconque des revendications 1 à 4, dans lequel le solvant est un éther.

6. Procédé selon l'une quelconque des revendications 1 à 4, dans lequel le solvant est le tétrahydrofuranne, le 1,2-diméthoxyéthane, le bis-(2-méthoxyéthyl)éther, l'éther, éthylique ou un mélange 20:1 en volume de tétrahydrofuranne et de 1,3-diméthylimidazolidinone.

7. Procédé selon l'une quelconque des revendications 1 à 6 dans lequel $R^1$ et $R^2$ représentent chacun, indépendammement l'un de l'autre, un atome d'hydrogène ou d'halogène.

8. Procédé selon la revendication 7, dans lequel $R^1$ est un atome d'hydrogène et $R^2$ est un atome d'halogène.

9. Procédé selon la revendication 8, dans lequel $R^1$ est un atome d'hydrogène et $R^2$ est un atome de fluor.

10. Procédé selon la revendication 9, dans lequel l'atome de fluor est en position *méta* par rapport à la position de fixation du radical (dicarboxyphényl)thio, sur le noyau gauche dans la formule I.

11. Procédé selon la revendication 7, dans lequel $R^1$ et $R^2$ sont tous les deux un atome d'hydrogène.

12. Procédé selon l'une quelconque des revendications 1 à 11, dans lequel on forme le dianion à partir d'un composé de formule III:

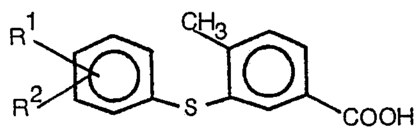

III

dans laquelle $R^1$ et $R^2$ sont tels que définis dans la revendication 1, d'abord par la formation d'un sel de carboxylate avec une base et ensuite par la déprotonation dans des conditions telles que celles metionnées dans l'une quelconque des revendications 2 à 6.

13. Procédé selon la revendication 12, dans lequel on effectue la déprotonation à l'aide d'une base de dialkylamide de métal alcalin, d'une base d'alkylamide ou d'une base d'amide de métal alcalin.

14. Procédé pour la préparation d'un composé dibenzo(b,f)thiépine, caractérisé en ce que l'on prépare un composé intermédiaire d'acide carboxyphénylthiophénylacétique de formule I mentionnée dans la revendication 1, selon un procédé tel que celui revendiqué dans l'une quelconque des revendications précédentes et qu'on le transforme ensuite par des moyens connus en la dibenzo(b,f)thiépine désirée.